(19) Europäisches Patentamt
European Patent Office
Office européen des brevets

(11) Veröffentlichungsnummer: **0 086 322 B1**

(12) **EUROPÄISCHE PATENTSCHRIFT**

(45) Veröffentlichungstag der Patentschrift: **01.06.88**

(51) Int. Cl.⁴: **C 07 D 307/60**, B 01 J 23/28, B 01 J 23/22

(21) Anmeldenummer: **83100055.9**

(22) Anmeldetag: **05.01.83**

(54) **Verfahren zur Herstellung von Ketopantolacton.**

(30) Priorität: **12.02.82 CH 889/82**

(43) Veröffentlichungstag der Anmeldung: **24.08.83 Patentblatt 83/34**

(45) Bekanntmachung des Hinweises auf die Patenterteilung: **01.06.88 Patentblatt 88/22**

(84) Benannte Vertragsstaaten: **AT BE CH DE FR GB IT LI NL**

(56) Entgegenhaltungen:
**DE - A - 2 243 583**
**DE - A - 2 403 042**

**BEILSTEINS HANDBUCH DER ORGANISCHEN CHEMIE, 4. Auflage, Band 17, Ergänzungswerke 3, 4 1975, SPRINGER-VERLAG, Berlin, Heidelberg, New York, Seite 5848, Zeilen 17-19**
**Chemical Abstracts Band 43, Nr. 18, 25. September 1949, Columbus, Ohio, USA S.H. LIPTON et al. "Synthesis of compounds related to pantothenic acid", Spalten 7426e-i - 7427a**
**TETRAHEDRON, Band 36, 1980, London G. DOLESCHALL et al. "The degradation of carboxylic acids into aldehydes. Regioselective alpha-acetoxylation of 1,2,4-triazolium salts with diacetoxyiodate(1)anion", Seiten 1649-1665**

(73) Patentinhaber: **F. HOFFMANN-LA ROCHE & CO. Aktiengesellschaft, CH-4002 Basel (CH)**

(72) Erfinder: **Nösberger, Paul, Dr., Passwangstrasse 1, CH-4127 Birsfelden (CH)**

(74) Vertreter: **Cottong, Norbert A. et al, Grenzacherstrasse 124 Postfach 3255, CH-4002 Basel (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft ein neues Verfahren zur Herstellung von Ketopantolacton (Dihydro-4,4-dimethyl-2,3-furandion), welches ein wichtiges Ausgangsmaterial für die Herstellung von optisch aktivem Pantolacton (Dihydro-3-hydroxy-4,4-dimethyl-2(3H)-furanon) darstellt.

Es sind bereits einige Verfahren zur Oxidation von racemischem Pantolacton zu Ketopantolacton bekannt, wobei bisher jedoch unter Verwendung relativ teurer Oxidationsmittel wie Bleitetraacetat, N-Bromsuccinimid oder Chromtrioxid (Jones-Reagens) nur relativ geringe Ausbeuten erzielt wurden. Eine Verbesserung dieses Verfahrens wurde durch Verwendung von Brom oder von Alkali- oder Erdalkalimetallhypochloriten in organischer Phase erreicht.

Weiterhin ist die Herstellung von Ketopantolacton aus Dimethylbrenztraubensäure und Formaldehyd bekannt. Dieses Verfahren hat jedoch den Nachteil, dass auch hier nur relativ geringe Ausbeuten erzielt werden, und dass das gewünschte Endprodukt auch nach Destillation in nicht ganz reiner Form anfällt.

Erfindungsgemäss wird nun Ketopantolacton der Formel

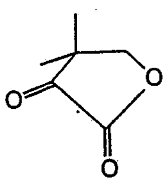

I

aus Pantolacton der Formel

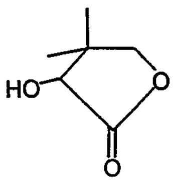

II

dadurch hergestellt, dass man Pantolacton der Formel II in Gegenwart eines Katalysators, welcher Vanadiumpentoxid, Molybdäntrioxid oder ein Vanadat oder Molybdat eines Übergangsmetalles oder ein Gemisch solcher Verbindungen enthält, mit Sauerstoff oder einem sauerstoffhaltigen Gas bei einer Temperatur von 150 °C bis 400 °C umsetzt.

Das erfindungsgemässe Verfahren stellt eine oxidative Dehydrierung von Pantolacton dar und führt in guter Ausbeute zum gewünschten Ketopantolacton. Da dieses Verfahren insbesondere auch mit Luft durchgeführt werden kann, wird der Einsatz teurer Oxidationsmittel vermieden. Zudem ist dieses Verfahren leicht in technischem Massstab anwendbar und kann insbesondere auch kontinuierlich durchgeführt werden.

Als weiterer Vorteil hat sich erwiesen, dass als Nebenprodukte hauptsächlich Wasser, Kohlendioxid und Kohlenmonoxid auftreten, so dass nach Abtrennung des Wassers und weiterer tiefsiedender Nebenprodukte direkt ein Produkt hoher Reinheit erhalten wird.

Ferner kann das erfindungsgemässe Verfahren auch mit technischem Pantolacton (Reinheit ca. 79–92%) durchgeführt werden. Es wurde sogar festgestellt, dass bei Verwendung von technischem Pantolacton häufig eine bessere Selektivität erreicht wird, und dass zusätzlich ein Reinigungseffekt erzielt wird, da ein Teil der Verunreinigungen bei dem Verfahren zerstört wird.

Die oxidative Dehydrierung von Pantolacton wird erfindungsgemäss in der Gasphase und in Gegenwart eines Katalysators durchgeführt, welcher Vanadiumpentoxid, Molybdäntrioxid oder ein Vanadat oder Molybdat eines Übergangsmetalles oder ein Gemisch solcher Verbindungen enthält. Bevorzugte Vandate und Molybdate sind diejenigen von Eisen, Nickel, Silber, Kupfer und Kobalt. Weiterhin können diese Katalysatoren gewünschtenfalls auch noch andere Metalle wie etwa Zinn, Antimon, Wismut oder Blei enthalten. Beispiele bevorzugter Katalysatoren sind Vanadiumpentoxid, Gemische von Vanadiumpentoxid oder Eisen-(III)-oxid mit Molybdäntrioxid, Eisen-silbermolybdat, Nickelvanadat, Kupfervanadat, Silbervanadat, Eisen-silbervanadat oder Kobaltvanadat. Besonders bevorzugt sind Katalysatoren, welche Vanadiumpentoxid und/oder Molybändtrioxid enthalten.

Der Katalysator kann in reiner Form vorliegen, mit einem inerten Trägermaterial gemischt sein oder auf einem inerten, geformten Trägermaterial fixiert sein. Beispiele inerter Trägermaterialien sind α-Aluminiumoxid, Keramik, Kieselguhr, Glas und Siliziumcarbid. Vorzugsweise werden Katalysatoren verwendet, die auf einem inerten Trägermaterial fixiert sind. Bevorzugte Trägermaterialien sind α-Aluminiumoxid und Keramik. Ganz besonders bevorzugt ist die Verwendung von Vanadiumpentoxid oder eines Gemisches von Vanadiumpentoxid und Molybdäntrioxid auf α-Aluminiumoxid als Katalysator.

Die erfindungsgemäss verwendeten Katalysatoren sind zum Teil im Handel erhältlich oder können leicht in an sich bekannter Weise hergestellt werden. Sie besitzen eine lange Lebensdauer und zeigen auch nach mehrmonatigem Gebrauch noch keinen Aktivitätsabfall. Ebenso bleibt die Selektivität der Katalysatoren konstant. Das erfindungsgemässe Verfahren kann daher vorteilhaft auch in einem Festbettreaktor durchgeführt werden.

Als Oxidationsmittel wird erfindungsgemäss Sauerstoff oder ein sauerstoffhaltiges Gas verwendet. Der Ausdruck «sauerstoffhaltiges Gas» bedeutet hierbei allgemein ein Gemisch von Sauerstoff und einem inerten Gas, wie beispielsweise Stickstoff, Kohlendioxid, Argon und Wasserdampf. Der Sauerstoffgehalt ist nicht kritisch. Im allgemeinen beträgt er jedoch etwa 1–40 Vol-% und vorzugsweise etwa 5–21 Vol-%. Besonders bevorzugt ist die Verwendung von Luft, der gewünschtenfalls zur Mässigung des Reaktionsverlaufs und zum Abführen von Reaktionswärme Stickstoff und/

oder Abgase aus dem Reaktor (nach Abtrennung von Ketopantolacton) zugesetzt werden können.

Die Menge Sauerstoff oder sauerstoffhaltiges Gas ist nach oben hin nicht kritisch. Um eine möglichst vollständige Umsetzung zu erreichen, sollte dem Reaktor pro Mol Pantolacton mindestens etwa 1 Mol Sauerstoff zugeführt werden. Mit Vorteil wird jedoch mit einem Überschuss an Sauerstoff gearbeitet, beispielsweise mit etwa 200–2000% und vorzugsweise mit etwa 300–1000% Überschuss.

Die erfindungsgemässe oxidative Dehydrierung von Pantolacton ist stark exotherm und temperaturabhängig. Bei zu hoher Temperatur kann die Totaloxidation zur Hauptreaktion werden, was zum Durchgehen des Reaktors führt. Die optimale Temperatur ist unabhängig vom verwendeten Katalysator, der Sauerstoff- und Pantolactonkonzentration im Eduktstrom, der Reinheit des Pantolactons, der Gasgeschwindigkeit sowie der Form und Grösse des Reaktors. Im allgemeinen wird in einem Temperaturbereich von etwa 150–400 °C, vorzugsweise etwa 200–350 °C gearbeitet. Besonders bevorzugt ist ein Temperaturbereich von etwa 250–300 °C.

Als Reaktormaterialien kommen grundsätzlich alle üblicherweise verwendeten Werkstoffe in Frage, welche Pantolacton und Ketopantolacton unter den Reaktionsbedingungen praktisch nicht angreifen, wie beispielsweise rostfreier Stahl, Glas und Keramik.

Die Katalysatormenge ist nicht kritisch. Die optimale Menge ist je nach Katalysator verschieden und zudem abhängig u. a. von der Temperatur, der Form und Grösse des Reaktors sowie der eingesetzten Menge Edukt. Die Optimierung wird zweckmässig so durchgeführt, dass der Katalysator und gewünschtenfalls inertes Trägermaterial in den Reaktor (vorzugsweise ein Festbettreaktor) eingefüllt werden und dann die Verweilzeit der Edukte und die Temperatur so eingestellt werden, dass der Umsatz an Pantolacton möglichst hoch ist, vorzugsweise über etwa 90%.

Das erfindungsgemässe Verfahren liefert das gewünschte Ketopantolacton nach Abtrennung des Wassers und der tiefsiedenden Nebenprodukte in einer Reinheit von ca. 99%. Die Trennung kann beispielsweise durch Abkühlen und gewünschtenfalls zusätzliches Auswaschen von Ketopantolacton mit Wasser erfolgen. Die Abgase können, wie oben erwähnt, gewünschtenfalls wieder dem Reaktor zugeführt werden. Gewünschtenfalls kann das erhaltene Produkt z.B. durch Kristallisation in Toluol oder Diäthyläther oder durch Rektifikation noch weiter gereinigt werden.

Das erfindungsgemässe Verfahren wird durch die folgenden Beispiele weiter veranschaulicht.

Beispiel 1

Der Reaktor besteht aus einem thermostatisierbaren Vorratsgefäss (95 °C) für Pantolacton, das an eine Dosierpumpe angeschlossen ist, einer Luftstromregelung, einem elektrisch geheizten Verdampferrohr (250 °C, Länge 20 cm) und einem Reaktionsrohr (Länge 60 cm, Durchmesser 3,5 cm), das von einem Luftbad (240 °C) umgeben ist und am oberen Ende mit dem Verdampferrohr verbunden ist. Die Rohrteile sind aus rostfreiem Stahl gefertigt. Als Katalysator wird mit 6% Vanadiumpentoxid und 3% Molybdäntrioxid belegtes α-Aluminiumoxid (erhältlich von Harshaw unter der Bezeichnung V-1002E) verwendet. Das Verdampferrohr sowie die obersten und untersten 10 cm des Reaktionsrohres werden mit Keramikkugeln gefüllt. Die restlichen 40 cm des Reaktionsrohres (Reaktionszone) werden unten (20 cm hoch) mit Katalysator, in der Mitte (10 cm hoch) mit Katalysator und Keramikkugeln im Verhältnis 1:1 und oben (10 cm hoch) mit Katalysator und Keramikkugeln im Verhältnis 1:3 bestückt. Dem Verdampfer werden nun stündlich 100 l Luft und 43 g Pantolacton zugeführt. Die Temperatur steigt an der heissesten Stelle in der Reaktionszone auf ca. 290 °C und beim Austritt aus der Reaktionszone auf ca. 250 °C. Hierbei werden 99,3% des Pantolactons umgesetzt, wobei mit einer Selektivität von 86,2% Ketopantolacton gebildet wird. Als Nebenprodukt erhält man vor allem Wasser und Kohlendioxid. Nach Abtrennung des Reaktionswassers und Spuren tiefsiedender Nebenprodukte am Rotationsverdampfer bei 60 °C und Wasserstrahlvakuum erhält man Ketopantolacton mit einer Reinheit von ca. 99%. Durch Umkristallisation in Toluol wird Ketopantolacton mit einer Reinheit von 99,9% erhalten; Smp. 67 °C, Sdp. 248 °C.

Beispiel 2

In dem in Beispiel 1 beschriebenen Reaktor wird als Katalysator mit 10% Vanadiumpentoxid belegtes α-Aluminiumoxid (V-0501S von Harshaw) eingesetzt und die Temperatur des Luftbades auf 245 °C eingestellt. Anschliessend werden dem Verdampfer stündlich 60 l Luft und 33 g Pantolacton zugeführt. Hierbei werden 99% des Pantolactons umgesetzt, wobei mit einer Selektivität von 85% Ketopantolacton gebildet wird.

Beispiel 3

In dem in Beispiel 1 beschriebenen Reaktor wird als Katalysator ein Gemisch von Eisen-(III)-oxid und Molybdäntrioxid (G-105 von Girdler) eingesetzt und die Temperatur des Luftbades auf 225 °C eingestellt. Anschliessend werden dem Verdampfer stündlich 60 l Luft und 33 g Pantolacton zugeführt. Hierbei werden 96% des Pantolactons umgesetzt, wobei mit einer Selektivität von 83% Ketopantolacton gebildet wird.

Beispiel 4

In dem in Beispiel 1 beschriebenen Reaktor wird als Katalysator ein Eisen-silbermolybdat auf Keramik eingesetzt und die Temperatur des Luftbades auf 245 °C eingestellt. Anschliessend werden dem Verdampfer stündlich 60 l Luft und 32 g Pantolacton zugeführt. Hierbei werden 97,8% des Pantolactons umgesetzt, wobei mit einer Selektivität von 78,7% Ketopantolacton gebildet wird.

Der verwendete Katalysator kann wie folgt hergestellt werden:

Eine Lösung von 24,72 g Ammoniumheptamolybdat-tetrahydrat in 500 ml Wasser wird unter gutem Rühren tropfenweise mit einer Lösung von 12,12 g Eisen-(III)-nitratnonahydrat und 5,10 g Silbernitrat in 100 ml Wasser versetzt. Der Niederschlag wird abgenutscht, mit Wasser gewaschen und dann zusammen mit 300 g Träger (Keramikkugeln mit Granulatbelag von 6 mm Durchmesser, erhältlich von Rosenthal unter der Bezeichnung RST Fl/Sp) in einen Weithalskolben gegeben und mit Wasser zugedeckt. Anschliessend wird das Wasser am Rotationsverdampfer unter möglichst langsamem Drehen bei 90 °C mit Hilfe eines Luftstromes entfernt. Der erhaltene Katalysator wird schliesslich noch 16 Stunden bei 450 °C ausgeheizt.

Beispiel 5

In dem in Beispiel 1 beschriebenen Reaktor wird als Katalysator Nickelvanadat auf Keramik eingesetzt und die Temperatur des Luftbades auf 270 °C eingestellt. Anschliessend werden dem Verdampfer stündlich 100 l Luft und 32 g Pantolacton zugeführt. Hierbei werden 99,2% des Pantolactons umgesetzt, wobei mit einer Selektivität von 79,8% Ketopantolacton gebildet wird.

Der verwendete Katalysator kann wie folgt hergestellt werden:

Zu einer 60 °C warmen Lösung von 13,66 g Ammoniumvanadat in 1000 ml Wasser wird unter gutem Rühren langsam eine Lösung von 16,98 g Nikkelnitrat in 50 ml Wasser zugegeben, dann am Rotationsverdampfer eingeengt und abgekühlt. Der erhaltene Niederschlag wird abgenutscht, gewaschen, analog zu Beispiel 4 auf Keramikkugeln fixiert und 16 Stunden bei 500 °C ausgeheizt.

Beispiel 6

In dem in Beispiel 1 beschriebenen Reaktor wird als Katalysator Kupfervanadat auf Keramik eingesetzt und die Temperatur des Luftbades auf 250 °C eingestellt. Anschliessend werden dem Verdampfer stündlich 60 l Luft und 32 g Pantolacton zugeführt. Hierbei werden 99,0% des Pantolactons umgesetzt, wobei mit einer Selektivität von 77,5% Ketopantolacton gebildet wird.

Der verwendete Katalysator kann wie folgt hergestellt werden:

Zu einer 60 °C warmen Lösung von 13,66 g Ammoniumvanadat in 1000 ml Wasser wird unter gutem Rühren langsam eine Lösung von 9,96 g Kupfer-(II)-chlorid-dihydrat in 50 ml Wasser zugegeben. Der erhaltene Niederschlag wird abgenutscht, gewaschen, analog zu Beispiel 4 auf Keramikkugeln fixiert und 16 Stunden bei 500 °C ausgeheizt.

Beispiel 7

In dem in Beispiel 1 beschriebenen Reaktor wird als Katalysator Silbervanadat auf Keramik eingesetzt und die Temperatur des Luftbades auf 250 °C eingestellt. Anschliessend werden dem Verdampfer stündlich 60 l Luft und 27 g Pantolacton zugeführt. Hierbei werden 98,4% des Pantolactons umgesetzt, wobei mit einer Selektivität von 84,4% Ketopantolacton gebildet wird.

Der verwendete Katalysator kann wie folgt hergestellt werden:

Zu einer auf 60 °C erwärmten Lösung von 8,78 g Ammoniumvanadat in 1000 ml Wasser wird unter gutem Rühren langsam eine Lösung von 15,5 g Silbernitrat in 125 ml Wasser zugegeben. Der erhaltene Niederschlag wird abgenutscht, gewaschen, analog zu Beispiel 4 auf Keramikkugeln fixiert und 16 Stunden bei 500 °C ausgeheizt.

Beispiel 8

In analoger Weise zu Beispiel 1 wird technisches Pantolacton (Gehalt 83,5%) umgesetzt. Die Temperatur des Luftbades wird auf 270 °C eingestellt und dem Verdampfer werden stündlich 60 l Luft und 41 g technisches Pantolacton zugeführt. Hierbei werden 99% des Pantolactons umgesetzt, wobei mit einer Selektivität von 92% (bezogen auf Pantolacton) Ketopantolacton gebildet wird.

**Patentansprüche**

1. Verfahren zur Herstellung von Ketopantolacton der Formel

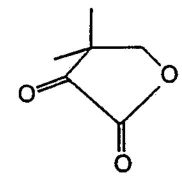

I

ams Pantolacton der Formel

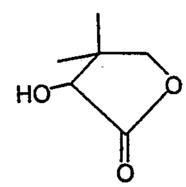

II

dadurch gekennzeichnet, dass man Pantolacton der Formel II in Gegenwart eines Katalysators, welcher Vanadiumpentoxid, Molybdäntrioxid oder ein Vanadat oder Molybdat eines Übergangsmetalles oder ein Gemisch solcher Verbindungen enthält, mit Sauerstoff oder einem sauerstoffhaltigen Gas bei einer Temperatur von 150 °C bis 400 °C umsetzt.

2. Verfahren nach Anspruch 1, dadurch gekennzeichnet, dass man als Vanadate oder Molybdate diejenigen von Eisen, Nickel, Silber, Kupfer oder Kobalt verwendet.

3. Verfahren nach Anspruch 1, dadurch gekennzeichnet, dass man einen Katalysator verwendet, der Vanadiumpentoxid oder Molybdäntrioxid enthält.

4. Verfahren nach einem der Ansprüche 1 bis 3, dadurch gekennzeichnet, dass man einen Katalysator verwendet, welcher auf einem inerten Trägermaterial fixiert ist.

5. Verfahren nach Anspruch 4, dadurch gekennzeichnet, dass man einen Katalysator verwendet, welcher auf α-Aluminiumoxid oder Keramik fixiert ist.

6. Verfahren nach Anspruch 5, dadurch gekennzeichnet, dass man als Katalysator Vanadiumpentoxid oder ein Gemisch von Vanadiumpentoxid und Molybdäntrioxid auf α-Aluminiumoxid verwendet.

7. Verfahren nach einem der Ansprüche 1 bis 6, dadurch gekennzeichnet, dass man die Reaktion mit Luft durchführt, welcher gewünschtenfalls noch Stickstoff und/oder Abgase aus dem Reaktor zugesetzt werden.

8. Verfahren nach einem der Ansprüche 1 bis 7, dadurch gekennzeichnet, dass man die Reaktion mit einem Überschuss an sauerstoffhaltigem Gas durchführt.

9. Verfahren nach einem der Ansprüche 1 bis 8, dadurch gekennzeichnet, dass man die Reaktion bei einer Temperatur von 200 °C bis 350 °C durchführt.

**Claims**

1. A process for the manufacture of ketopantolactone of the formula

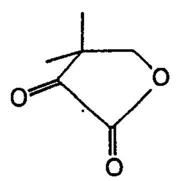

I

from pantolactone of the formula

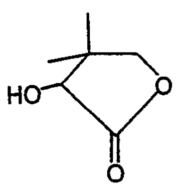

II ,

characterized by reacting pantolactone of formula II in the presence of a catalyst, which contains vanadium pentoxide, molybdenum trioxide or a vanadate or molybdate of a transition metal or a mixture of such compounds, with oxygen or an oxygen-containing gas at a temperature of 150 °C to 400 °C.

2. A process according to claim 1, characterized in that a vanadate or molybdate of iron, nickel, silver, copper or cobalt is used as the vanadate or molybdate.

3. A process according to claim 1, characterized in that a catalyst which contains vanadium pentoxide or molybdenum trioxide is used.

4. A process according to any one of claims 1 to 3, characterized in that a catalyst which is fixed on an inert carrier material is used.

5. A process according to claim 4, characterized in that a catalyst which is fixed on α-aluminium oxide or ceramics is used.

6. A process according to claim 5, characterized in that vanadium pentoxide or a mixture of vanadium pentoxide and molybdenum trioxide on α-aluminium oxide is used as the catalyst.

7. A process according to any one of claims 1 to 6, characterized in that the reaction is carried out with air to which, if desired, is/are added nitrogen and/or exhaust gases from the reactor.

8. A process according to any one of claims 1 to 7, characterized in that the reaction is carried out with an excess of oxygen-containing gas.

9. A process according to any one of claims 1 to 8, characterized in that the reaction is carried out at a temperature of 200 °C to 350 °C.

**Revendications**

1. Procédé de préparation de la cétopantolactone de formule

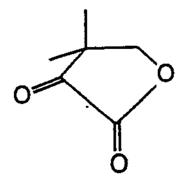

I

à partir de la pantolactone de formule

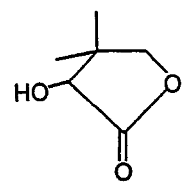

II

caractérisé en ce que l'on fait réagir la pantolactone de formule II, en présence d'un catalyseur contenant du pentoxyde de vanadium, du trioxyde de molybdène ou un vanadate ou molybdate d'un métal de transition ou un mélange de ces composés, avec l'oxygène ou un gaz contenant de l'oxygène à une température de 150 à 400 °C.

2. Procédé selon la revendication 1, caractérisé en ce que l'on utilise en tant que vanadates ou molybdates les vanadates ou molybdates du fer, du nickel, de l'argent, du cuivre ou du cobalt.

3. Procédé selon la revendication 1, caractérisé en ce que l'on utilise un catalyseur contenant du pentoxyde de vanadium ou du trioxyde de molybdène.

4. Procédé selon l'une des revendications 1 à 3, caractérisé en ce que l'on utilise un catalyseur fixé sur une matière de support inerte.

5. Procédé selon la revendication 4, caractérisé en ce que l'on utilise un catalyseur fixé sur α-alumine ou sur une matière céramique.

6. Procédé selon la revendication 5, caractérisé en ce que l'on utilise en tant que catalyseur du pentoxyde de vanadium ou un mélange de pentoxyde de vanadium et de trioxyde de molybdène sur α-alumine.

7. Procédé selon l'une des revendications 1 à 6, caractérisé en ce que l'on effectue la réaction avec de l'air, auquel on ajoute encore si on le désire de l'azote et/ou des gaz résiduaires provenant du réacteur.

8. Procédé selon l'une des revendications 1 à 7, caractérisé en ce que l'on effectue la réaction avec un excès de gaz contenant de l'oxygène.

9. Procédé selon l'une des revendications 1 à 8, caractérisé en ce que l'on effectue la réaction à une température de 200 à 350 °C.